# EUROPEAN PATENT SPECIFICATION

(11) **EP 4 582 050 B1**
(45) Date of publication and mention of the grant of the patent: **13.05.2026**
(21) Application number: 25150246.4
(22) Date of filing: 03.01.2025
(51) Int. Cl.: A61C 7/00, G16H 50/50

(54) **METHOD, COMPUTING DEVICE AND COMPUTER PROGRAM FOR PROVIDING SOLUTION FOR ESTABLISHING ORTHODONTIC PLANNING USING 3D TOOTH MODEL**
VERFAHREN, RECHNERVORRICHTUNG UND COMPUTERPROGRAMM ZUR BEREITSTELLUNG EINER LÖSUNG ZUR HERSTELLUNG EINER ORTHODONTISCHEN PLANUNG UNTER VERWENDUNG EINES 3D-ZAHNMODELLS
PROCÉDÉ, DISPOSITIF INFORMATIQUE ET PROGRAMME INFORMATIQUE POUR FOURNIR UNE SOLUTION POUR ÉTABLIR UNE PLANIFICATION ORTHODONTIQUE À L'AIDE D'UN MODÈLE DENTAIRE 3D

(30) Priority: 04.01.2024 KR 20240001475
(43) Date of publication of application: 09.07.2025
(73) Proprietor: 3D ONS, INC., Seoul 06023 (KR)
(72) Inventor: CHO, Heonjae, Seoul (KR); OH, Seungyoung, Seoul (KR)
(74) Representative: Walaski, Jan Filip

(56) References cited:
- WO-A1-2018/118200
- WO-A1-2023/220837
- US-A1- 2005 208 449
- US-A1- 2007 168 152
- US-A1- 2022 211 467
- US-A1- 2023 005 593
- US-A1- 2023 080 308

## Description

### BACKGROUND

### 1. Field of the Invention

The present invention relates to a method, computing device, and computer program for providing a solution for establishing orthodontic planning using a 3D tooth model.

### 2. Discussion of Related Art

In general, uneven dentition or uneven malocclusion of teeth may cause abnormal development of teeth themselves, abnormal development of a jawbone, etc. People with uneven dentition or malocclusion cover their mouths when talking or laughing with others due to the dentition, and may also become passive in interpersonal relationships, making it difficult to lead a satisfactory social life.

In addition, when eating food, food may not be ground evenly and may get stuck between teeth, which may cause various dental diseases or diseases of the digestive system.

Therefore, in order to solve this problem, an orthodontic technology is being applied that causes the movement of teeth along with the remodeling of an alveolar bone surrounding the teeth by continuously applying force to the teeth.

Meanwhile, systematic knowledge of the movement of teeth is required during orthodontic treatment, an accurate dental laboratory process should be established as the basis, and systematic monitoring is essential in the actual clinical process.

In the past, various programs for orthodontic treatment were developed, and 3D tooth models were created and analyzed through these programs to more accurately establish the orthodontic planning.

However, the movement of teeth includes linear and rotational movements, and in order to plan the linear and rotational movements more accurately, it is important to accurately set the axis and center of rotation of a tooth, and therefore information on a root is very important to more accurately establish orthodontic planning. However, because conventional orthodontic programs establish orthodontic planning by considering only a crown area of a tooth, it is difficult to establish accurate orthodontic planning. US 2007/0168152 A1 discloses acquiring a digital model of a patient's teeth, automatically detecting reference data or features based on the digital model, and automatically computing dental measurements based on said reference data or features. US 2023/080308 A1 discloses a method and system for optimizing dental aligner geometry. US 2023/005593 A1 discloses systems and methods for manufacturing an orthodontic appliance. WO 2018/118200 A1 discloses orthodontic planning systems. WO 2023/220837 A1 discloses a system and method for orthodontic appliance delivery. US 2005/208449 A1 discloses root-based tooth moving sequencing. US 2022/211467 A1 discloses orthodontic attachment systems and methods.

### SUMMARY OF THE INVENTION

The present invention is directed to providing a method, a computing device, and computer program for providing a solution for establishing orthodontic planning using a 3D tooth model capable of more accurately establishing the orthodontic planning by generating the 3D tooth model by performing accurate modeling on not only a crown area but also a root area and establishing the orthodontic planning using the generated 3D tooth model.

The problems to be solved by the present invention are not limited to the above-described problems, and other problems that are not described may be obviously understood by those skilled in the art from the following description.

According to the present invention, there is provided a method of providing a solution for establishing orthodontic planning using a 3D tooth model by a computing device according to claim 1, a computing device according to claim 11, and a computer program stored on a computer device-reading recording medium coupled to the computing device, according to claim 12. Preferred features are set out in the dependent claims.

Other detailed contents of the present invention are described in a detailed description and are illustrated in the drawings.

### BRIEF DESCRIPTION OF DRAWINGS

The above and other objects, features and advantages of the present invention will become more apparent to those of ordinary skill in the art by describing exemplary embodiments thereof in detail with reference to the accompanying drawings, in which:
FIG. 1 is a diagram illustrating a system for providing a solution for establishing orthodontic planning using a 3D tooth model according to an embodiment of the present invention;
FIG. 2 is a diagram for describing a hardware configuration of a computing device according to another embodiment of the present invention;
FIG. 3 is a flowchart of a method of providing a solution for establishing orthodontic planning using a 3D tooth model according to another embodiment of the present invention;
FIG. 4 is a flowchart for describing a method of generating a 3D tooth model in various embodiments;
FIGS. 5A and 5B are diagrams illustrating tooth scan data and tooth computed tomography (CT) data used for generating a 3D tooth model in various embodiments; and
FIGS. 6A and 6B are diagrams for exemplarily illustrating a form in which the tooth scan data and the tooth CT data are aligned in various embodiments.

### DETAILED DESCRIPTION OF EXEMPLARY EMBODIMENTS

Advantages and features of the present invention and methods to achieve them will be elucidated from exemplary embodiments described below in detail with reference to the accompanying drawings. However, the present invention is not limited to embodiments to be described below, but may be implemented in various different forms, these embodiments will be provided only in order to make the present invention complete and allow those skilled in the art to completely recognize the scope of the present invention, and the present invention will be defined by the scope of the claims.

The terms as used herein are for describing exemplary embodiments rather than limiting the present invention. In the present specification, a singular form includes a plural form unless stated otherwise in the phrase. The terms "comprise" and/or "comprising" used in the present invention do not exclude the existence or addition of one or more other components other than the mentioned components.

Like reference numerals refer to like components throughout the specification and "and/or" includes each of the components mentioned and all combinations thereof. The terms "first," "second," and the like are used to describe various components, but these components are not limited by these terms. These terms are used only in order to distinguish one component from other components.

Further, the term "unit" or "module" used herein means a hardware component such as software, an FPGA, or an ASIC and performs predetermined functions. However, the term "unit" or "module" is not meant to be limited to software or hardware. A "unit" or "module" may be configured to be stored in a storage medium that can be addressed or may be configured to regenerate one or more processors. Accordingly, for example, a "unit" or "module" includes components such as software components, object-oriented software components, class components, and task components, processors, functions, attributes, procedures, subroutines, segments of a program code, drivers, firmware, a microcode, a circuit, data, a database, data structures, tables, arrays, and variables. Functions provided in components, "units," or "modules" may be combined into fewer components, "units," or "modules" or further separated into additional components, "units," or "modules."

Spatially relative terms "below," "beneath," "lower," "above," "upper," and the like, may be used in order to easily describe correlations between one component and other components. The spatially relative terms should be understood as terms including different directions of components during use or operation in addition to the directions illustrated in the drawings. For example, when components illustrated in the drawings are flipped, a component described as "below" or "beneath" another component may be placed "above" the other component. Accordingly, an illustrative term "below" may include both of a downward direction and an upward direction. Components may be oriented in other directions as well, and thus, spatially relative terms may be interpreted according to orientations.

Unless the context dictates otherwise, expressions such as "first," "second," "1^{st}," or "2^{nd}" used in this document are used to distinguish one object from another when referring to a plurality of objects of the same type and do not limit the order or importance of the objects in question.

As used herein, the expressions "A, B, and C," "A, B, or C," "A, B, and/or C," "at least one of A, B, and C," "at least one of A, B, or C," "at least one of A, B, and/or C," "at least one selected from A, B, and C," "at least one selected from A, B, or C," "at least one selected from A, B, and/or C," etc., may mean each of the listed items or all possible combinations of the listed items. For example, "at least one selected from A and B" may refer to (1) A, (2) at least one A, (3) B, (4) at least one B, (5) at least one A and at least one B, (6) at least one of A and B, (7) at least one of B and A, (8) both A and B.

The expression "based on" is used herein to describe one or more factors affecting the decision, act of judgment, or action described in the phrase or sentence containing the expression, and this expression does not exclude additional factors influencing the decision, or act or action of judgment.

As used herein, a component (e.g., a first component) being "connected" or "coupled" to another component (e.g., a second component) may mean not only that the component is directly connected or coupled to the other component, but also that it is connected or coupled to other component via another component (e.g., a third component).

As used herein, the expression "configured to" may mean "set to," "having the ability to," "modified to," "made to," "capable of," etc., according to the context. The corresponding expression is not limited to the meaning of "specifically designed in hardware." For example, a processor configured to perform a specific operation may be a generic-purpose processor that can perform the specific operation by executing software.

Unless defined otherwise, all terms (including technical and scientific terms) used in the present invention have the same meaning as commonly understood by those skilled in the art to which the present invention pertains. In addition, terms defined in generally used dictionaries are not ideally or excessively interpreted unless they are specifically defined clearly.

In this specification, a computer is any kind of hardware device including at least one processor, and can be understood as including a software configuration which is operated in the corresponding hardware device according to the embodiment. For example, the meaning of "computer" may be understood to include all of smart phones, tablet PCs, desktops, laptops, and user clients and applications running on each device, but is not limited thereto.

Hereinafter, embodiments of the present invention will be described in detail with reference to the accompanying drawings.

Each step described in this specification is described as being performed by the computer, but subjects of each step are not limited thereto, and according to embodiments, at least some of each step can also be performed on different devices.

Orthodontic treatment means moving teeth, more specifically, a root between bones (e.g., jawbone, skull).

A periodontal ligament (e.g., cells, collagen fibers, blood vessels, tissue fluid, etc.) with a thickness of about 0.5 mm exists between the root (hard tissue) and the bone (hard tissue). Surrounding periodontal ligaments are alive as blood flows through the blood vessels included in the periodontal ligament.

In this case, when applying force to a tooth for orthodontic treatment, such as wearing braces, the force is transmitted to the root as the tooth is pushed out, and the root moves due to this force. In this process, the periodontal ligament existing between the root and the bone on the side where the root moved is reduced from 0.5 mm to about 0.2 mm, and the side opposite to the direction where the root moves is increased by that amount.

In this way, when the thickness of the periodontal ligament decreases, the blood vessels become narrower, the blood flow decreases, and the amount of oxygen supplied to the periodontal ligament becomes insufficient. Accordingly, osteoclasts are created to absorb the surrounding roots and bones in order to restore the periodontal ligament to its original thickness.

In this process, since the bone is absorbed faster than the root, the bone is reduced and space is secured, and flesh is restored again. This process is repeatedly performed to move the tooth to a target position.

Meanwhile, the blood vessels in the area of the periodontal ligament that has the reduced thickness are subjected to compression accordingly, and when the thickness of the blood vessels becomes less than or equal to 0.2 mm, the blood vessels are completely compressed and thus blood does not flow. Because the blood flowing through the blood vessels does not flow when the thickness of the blood vessels becomes less than or equal to 0.2 mm, this process is not repeatedly performed, and the surrounding osteoclasts die, causing teeth to not move.

Therefore, in the orthodontic treatment, it is very important to precisely control the amount of movement of the root so that the thickness of the periodontal ligament may be compressed only to a minimum of 0.2 mm to 0.25 mm.

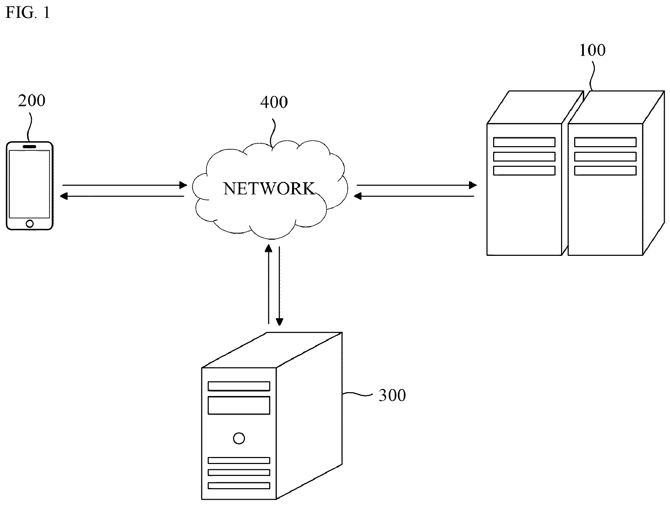

Accordingly, the method, computing device, and computer program for providing a solution for establishing orthodontic planning using a 3D tooth model according to various embodiments of the present invention provide a solution that enables the 3D tooth model to be more accurately implemented based on anatomical information of not only the crown but also the root, and the orthodontic planning to be established based on this 3D tooth model, thereby establishing the orthodontic planning that can precisely control the amount of movement of the root. Hereinafter, a description will be provided in detail with reference to FIGS. 1 to 6.

FIG. 1 is a diagram illustrating a system for providing a solution for establishing orthodontic planning using a 3D tooth model according to an embodiment of the present invention.

Referring to FIG. 1, a system for providing a solution for establishing orthodontic planning using a 3D tooth model according to an embodiment of the present invention may include a computing device 100, a user terminal 200, an external server 300, and a network 400.

Here, the system for providing a solution for establishing orthodontic planning using a 3D tooth model illustrated in FIG. 1 is according to an embodiment, and its components are not limited to the embodiment illustrated in FIG. 1, and may be added, changed, or deleted as needed.

In an embodiment, the computing device 100 may provide a solution for establishing orthodontic planning using a 3D tooth model.

In various embodiments, the computing device 100 may generate a 3D tooth model for a subject requiring orthodontic treatment, and provide a solution for establishing orthodontic planning based on the 3D tooth model.

Here, the solution for establishing orthodontic planning may be to provide information for establishing the orthodontic planning based on the 3D tooth model, guide the orthodontic planning based on the information for establishing the orthodontic planning or automatically establish the orthodontic planning, but is not limited thereto.

In various embodiments, the computing device 100 may be connected to a user terminal 200 via a network 400, and can provide a service for providing a solution for establishing orthodontic planning using a 3D tooth model to the user terminal 200. For example, the computing device 100 may provide the information for establishing the orthodontic planning to the user terminal 200 in response to a solution provision request acquired from the user terminal 200, or may provide guide information for guiding the establishment of the orthodontic planning based on the information for establishing the orthodontic planning.

Here, the user terminal 200 may be any type of entity(s) in the system that has a mechanism for communication with the computing device 100. For example, the user terminal 200 may include a personal computer (PC), a notebook, a mobile terminal, a smart phone, a tablet PC, a wearable device, etc., and may include all types of terminals that may access wired/wireless networks. In addition, the user terminal 200 may include any computing device implemented by at least one of an agent, an application programming interface (API), and a plug-in. In addition, the user terminal 200 may include an application source and/or a client application.

In addition, here, the network 400 may be a connection structure capable of exchanging information between respective nodes such as a plurality of terminals and servers. For example, the network 400 may include a local area network (LAN), a wide area network (WAN), the Internet (World Wide Web (WWW)), a wired/wireless data communication network, a telephone network, a wired/wireless television communication network, a controller area network (CAN), Ethernet, or the like.

Examples of the wireless data communication network may include 3G, 4G, 5G, 3rd Generation Partnership Project (3GPP), 5th Generation Partnership Project (5GPP), Long Term Evolution (LTE), World Interoperability for Microwave Access (WiMAX), Wi-Fi, Internet, a local area network (LAN), a wireless local area network (WLAN), a wide area network (WAN), a personal area network (PAN), radio frequency, a Bluetooth network, a near-field communication (NFC) network, a satellite broadcast network, an analog broadcast network, a digital multimedia broadcasting (DMB) network, and the like, but are not limited thereto.

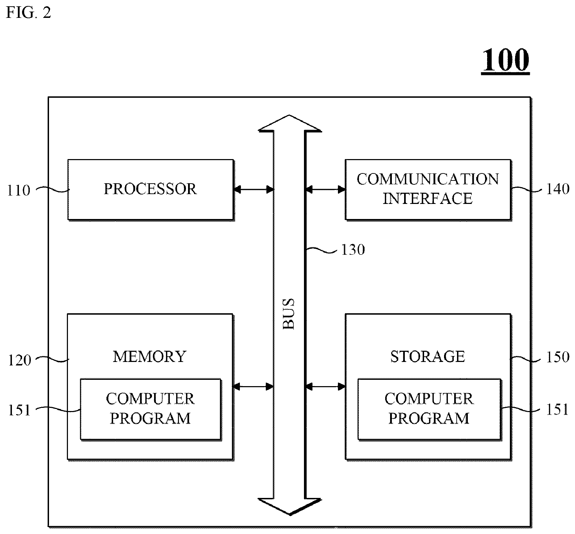

In an embodiment, the external server 300 may be connected to the computing device 100 through the network 400, and may store and manage information and data required for the computing device 100 to provide various services, or may collect, store, and manage information and data generated as the computing device 100 performs various services. For example, the external server 300 may be a storage server separately provided outside the computing device 100, but is not limited thereto. Hereinafter, a hardware configuration of the computing device 100 will be described with reference to FIG. 2.

FIG. 2 is a diagram for describing a hardware configuration of a computing device according to another embodiment of the present invention.

Referring to FIG. 2, the computing device 100 according to another embodiment of the present invention may include one or more processors 110, a memory 120 that loads a computer program 151 executed by the processor 110, a bus 130, a communication interface 140, and a storage 150 that stores the computer program 151. Here, only the components related to the embodiment of the present invention are illustrated in FIG. 2. Accordingly, one of ordinary skill in the art to which the present invention pertains may know that the computing device 210 may further include other general-purpose components in addition to the components illustrated in FIG. 2.

The processor 110 controls an overall operation of each component of the computing device 100. The processor 110 may be configured to include a central processing unit (CPU), a micro processor unit (MPU), a micro controller unit (MCU), a graphics processing unit (GPU), or any type of processor well known in the art of the present invention.

In addition, the processor 110 may perform an operation on at least one application or program for executing the method according to the embodiments of the present invention, and the computing device 100 may include one or more processors.

In various embodiments, the processor 110 may further include a random access memory (RAM) (not illustrated) and a read-only memory (ROM) (not illustrated) for temporarily and/or permanently storing signals (or data) processed in the processor 110. In addition, the processor 110 may be implemented in the form of a system on chip (SoC) including at least one of a GPU, a RAM, and a ROM.

The memory 120 stores various data, commands, and/or information. The memory 120 may load the computer program 150 from the storage 150 to execute methods/operations according to various embodiments of the present invention. When the computer program 151 is loaded into the memory 120, the processor 110 may perform the method/operation by executing one or more instructions constituting the computer program 151. The memory 120 may be implemented as a volatile memory such as a random access memory (RAM), but the technical scope of the present invention is not limited thereto.

The bus 130 provides a communication function between the components of computing device 100. The bus 130 may be implemented as various types of buses, such as an address bus, a data bus, and a control bus.

The communication interface 140 supports wired/wireless Internet communication of the computing device 100. In addition, the communication interface 140 may support various communication manners other than the Internet communication. To this end, the communication interface 140 may be configured to include a communication module well known in the art of the present invention. In some embodiments, the communication interface 140 may be omitted.

The storage 150 may non-temporarily store the computer program 151. When performing a process of providing a solution for establishing orthodontic planning using a 3D tooth model through the computing device 100, the storage 150 may store various types of information necessary for providing a process of providing a solution for establishing orthodontic planning using a 3D tooth model.

The storage 150 may include a nonvolatile memory, such as a ROM, an erasable programmable ROM (EPROM), an electrically erasable programmable ROM (EEPROM), and a flash memory, a hard disk, a removable disk, or any well-known computer-readable recording medium in the art to which the present invention pertains.

The computer program 151 may include one or more instructions to cause the processor 110 to perform methods/operations according to various embodiments of the present invention when loaded into the memory 120. That is, the processor 110 may perform the method/operation according to various embodiments of the present invention by executing the one or more instructions.

In an embodiment, the computer program 151 may include one or more instructions for performing a method of providing a solution for establishing orthodontic planning using a 3D tooth model, the method including generating the 3D tooth model including a plurality of individual tooth models generated by individually modeling each of a plurality of teeth for a subject, and providing a solution for establishing orthodontic planning based on the generated 3D tooth model.

Operations of the method or algorithm described with reference to the embodiment of the present invention may be directly implemented in hardware, in software modules executed by hardware, or in a combination thereof. The software module may reside on a random access memory (RAM), a read only memory (ROM), an erasable programmable ROM (EPROM), an electrically erasable programmable ROM (EEPROM), a flash memory, a hard disk, a removable disk, or a compact disk (CD)-ROM, or may reside on any type of computer readable recording medium well-known in the art.

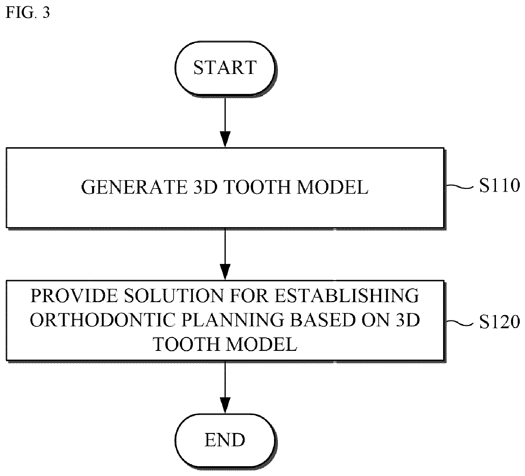

The components of the present invention may be embodied as a program (or application) and stored in a medium for execution in combination with a computer which is hardware. The components of the present invention may be executed in software programming or software elements, and similarly, embodiments may be realized in a programming or scripting language such as C, C++, Java, and an assembler, including various algorithms implemented in a combination of data structures, processes, routines, or other programming constructions. Functional aspects may be implemented in algorithms executed on one or more processors. Hereinafter, a method of providing a solution for establishing orthodontic planning using a 3D tooth model performed by the computing device 100 will be described with reference to FIGS. 3 to 6.

FIG. 3 is a flowchart of a method of providing a solution for establishing orthodontic planning using a 3D tooth model according to another embodiment of the present invention.

Referring to FIG. 3, in operation S110, the computing device 100 can generate a 3D tooth model for teeth of a subject based on anatomical information about a crown and a root of the teeth.

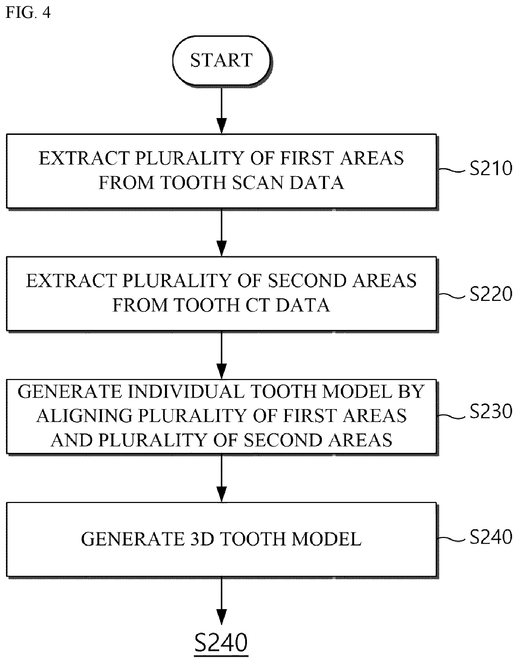

In various embodiments, the computing device 100 may generate a 3D tooth model including a plurality of individual tooth models by individually modeling each of the plurality of teeth based on data corresponding to the plurality of teeth for the target. Here, the method of generating a 3D tooth model performed by the computing device 100 will be described later with reference to FIGS. 4 to 6.

In operation S120, the computing device 100 may provide the solution for establishing orthodontic planning based on the 3D tooth model generated through operation S110.

In various embodiments, the computing device 100 may provide results of reviewing orthodontic planning set by a user as the solution for establishing orthodontic planning.

More specifically, first, when correction is required for at least one of a plurality of teeth, the computing device 100 may establish temporary orthodontic planning for an orthodontic tooth based on a crown area of an individual tooth model corresponding to at least one tooth requiring correction (hereinafter referred to as "orthodontic tooth").

Here, the orthodontic tooth may be a tooth selected by a user who wishes to establish orthodontic planning among the plurality of teeth. However, in some cases, the orthodontic tooth may be a tooth spaced a predetermined length from an arch line corresponding to the plurality of teeth, and may be determined by analyzing data (e.g., tooth scan data and/or tooth CT data, etc.) generated by photographing the plurality of teeth.

In addition, the temporary orthodontic planning may include, but is not limited to, information on the orthodontic tooth, the target position of the orthodontic tooth, and information on the amount of unit orthodontic movement for the orthodontic tooth.

In addition, here, the amount of unit orthodontic movement may include, but is not limited to, a unit movement amount (e.g., 0.3 mm) and a unit rotation amount (e.g., 1°) in a direction based on at least one of an X-axis, a Y-axis, and a Z-axis.

Thereafter, the computing device 100 may review the temporary orthodontic planning based on the root area of the individual tooth model corresponding to the orthodontic tooth.

For example, the computing device 100 may calculate the expected amount of orthodontic movement of the root corresponding to the amount of unit orthodontic movement of the orthodontic tooth according to the temporary orthodontic planning, and may review whether the expected amount of orthodontic movement of the root is greater than or equal to a threshold value.

For example, the computing device 100 may set an axis (actual axis of a tooth and a center of movement) in a longitudinal direction on the root area of an individual tooth model for an orthodontic tooth, and may calculate an expected amount of orthodontic movement of a root corresponding to an amount of unit orthodontic movement of the orthodontic tooth by using the axis and center of movement set on the root area of the individual tooth model for the orthodontic tooth.

Here, the method of setting the center of movement on the root area may be set based on at least one of a first method of setting the center of movement based on a user input acquired through a user interface (UI) that outputs a 3D tooth model, and a second method of automatically setting the center of movement based on a landmark (e.g., a root apex), a center of the tooth, and a gingival apex (crest of the alveolar bone), etc.) located on the root area of the individual tooth model.

For example, the computing device 100 may set the center of movement on the root area of the individual tooth model based on the first method or the second method.

In addition, the computing device 100 may set the center of movement on the root area of the individual tooth model based on the first method and verify the center of movement set by the first method based on the second method.

In addition, the computing device 100 may set the center of movement on the root area of the individual tooth model based on the first method and verify the center of movement set by the first method based on the second method.

As another example, the computing device 100 may calculate the expected amount of orthodontic movement of the root corresponding to the amount of unit orthodontic movement of the orthodontic tooth according to the temporary orthodontic planning, and review whether a thickness of a periodontal ligament between the root area of the orthodontic tooth and the bone is less than a critical thickness based on the expected amount of orthodontic movement of the root. For example, the computing device 100 may determine an expected movement direction and an expected movement amount of a root area of a specific tooth when a crown area of the specific tooth moves in a specific direction, determine a location of the root area of the specific tooth based on the expected movement direction and the expected movement amount of the root area of the specific tooth, and review whether a length of a periodontal ligament existing between the root area of the specific tooth at the determined location and the bone located in the expected movement direction of the specific tooth is less than a critical length (e.g., 0.2 mm).

As another example, the computing device 100 may review whether the orthodontic period corresponding to the amount of unit orthodontic movement of the orthodontic tooth according to the temporary orthodontic planning is within the allowable orthodontic range. For example, the computing device 100 may calculate the orthodontic amount of root area relative to the orthodontic amount of crown area for each of the plurality of individual tooth models based on the 3D tooth model, calculate the orthodontic period for the orthodontic tooth based on the orthodontic amount of root area relative to the orthodontic amount of crown area, and review whether the calculated orthodontic period is within the allowable orthodontic range.

Here, the allowable orthodontic range is a range defining an allowable orthodontic period. For example, the allowable orthodontic range may be a range from an orthodontic period expected to be required when the tooth is corrected according to the maximum amount of correction to an orthodontic period expected to be required when the tooth is corrected according to the minimum amount of correction.

In this case, the allowable correction range is determined based on the maximum/minimum correction amount of tooth, and since the maximum/minimum correction amount may be set differently for each tooth, the allowable correction range may also be set individually for each tooth.

In various embodiments, when the correction from the first point to the second point is required for a specific tooth, the computing device 100 may divide the section from the first point to the second point based on the amount of unit orthodontic movement of the specific tooth according to the temporary orthodontic planning, and may calculate the orthodontic period for at least one tooth based on the number of divided sections. However, the present invention is not limited thereto.

Thereafter, the computing device 100 may provide a solution for establishing final orthodontic planning based on the review result for the temporary orthodontic planning.

For example, when the expected amount of orthodontic movement for the root area of the orthodontic tooth is less than a threshold value, the computing device 100 may provide the information on the expected amount of orthodontic movement for the root area of the orthodontic tooth.

In this case, when the expected amount of orthodontic movement for the root area of the orthodontic tooth is greater than or equal to a threshold value, the computing device 100 may provide information on a first adjustment value corresponding to the amount of unit orthodontic movement of the orthodontic tooth, provide guide information for guiding adjustment of the amount of unit orthodontic movement of the orthodontic tooth by the first adjustment value, or automatically adjust the amount of unit orthodontic movement of the orthodontic tooth by the first adjustment value.

Here, the first adjustment value may be, but is not limited to, a difference value between the amount of unit orthodontic movement that makes the expected amount of orthodontic movement of the root area of the orthodontic tooth less than the threshold value and the amount of unit orthodontic movement of the orthodontic tooth according to the temporary orthodontic planning.

As another example, when the thickness of the periodontal ligament between the root area of the orthodontic tooth and the bone is determined to be more than or equal to the critical thickness, the computing device 100 may provide the information on the thickness of the periodontal ligament.

In this case, when the thickness of the periodontal ligament between the root area of the orthodontic tooth and the bone is determined to be less than the critical thickness, the computing device 100 may provide information on a second adjustment value corresponding to the amount of unit orthodontic movement of the orthodontic tooth, provide guide information for guiding adjustment of the amount of unit orthodontic movement of the orthodontic tooth by the second adjustment value, or automatically adjust the amount of unit orthodontic movement of the orthodontic tooth by the second adjustment value.

Here, the second adjustment value may be, but is not limited to, the difference value between the amount of unit orthodontic movement that makes the thickness of the periodontal ligament between the root area of the orthodontic tooth and the bone more than or equal to the critical thickness and the amount of unit orthodontic movement of the orthodontic tooth according to the temporary orthodontic planning.

As another example, the computing device 100 may provide the information on the orthodontic period when the orthodontic period for the orthodontic tooth falls within the allowable orthodontic range.

In this case, when the orthodontic period for the orthodontic teeth is outside the allowable orthodontic range, the computing device 100 may provide information on a third adjustment value corresponding to the amount of unit orthodontic movement of the orthodontic tooth, provide guide information for guiding the adjustment of the amount of unit orthodontic movement of the orthodontic tooth by the third adjustment value, or automatically adjust the amount of unit orthodontic movement of the orthodontic tooth by the third adjustment value.

Here, the third adjustment value may be, but is not limited to, a difference value between the amount of unit orthodontic movement that makes the orthodontic period of the orthodontic tooth fall within the allowable orthodontic range and the amount of unit orthodontic movement of the orthodontic tooth according to the temporary orthodontic planning.

In various embodiments, when rapid orthodontic correction is required for a specific tooth based on the state of the subject, the computing device 100 may guide the adjustment of the amount of unit orthodontic movement of the specific tooth to the correction amount corresponding to the minimum value of the allowable orthodontic range, or may automatically adjust the amount of unit orthodontic movement of the specific tooth to the correction amount corresponding to the minimum value of the allowable orthodontic range.

Meanwhile, when rapid orthodontic correction is required for a specific tooth based on the state of the subject, the computing device 100 may guide the adjustment of the amount of unit orthodontic movement of the specific tooth to the correction amount corresponding to the maximum value of the allowable orthodontic range, or may automatically adjust the amount of unit orthodontic movement of the specific tooth to the correction amount corresponding to the maximum value of the allowable orthodontic range. However, the present invention is not limited thereto.

In various embodiments, the computing device 100 may provide information on optimal orthodontic planning as the solution for establishing orthodontic planning.

More specifically, first, when movement from a current position to a target position is required for an orthodontic tooth, the computing device 100 may determine the amount of unit orthodontic movement for the orthodontic tooth using the individual tooth model corresponding to the orthodontic tooth. For example, when the movement to the target position is required for the specific tooth, the computing device 100 may determine the amount of unit orthodontic movement of the specific tooth so that the expected amount of orthodontic movement of the root area of the specific tooth is less than the threshold value based on the individual tooth model corresponding to the specific tooth, determine the amount of unit orthodontic movement of the specific tooth so that the thickness of the periodontal ligament between the root area of the specific tooth and the bone located in the direction in which the specific tooth moves is greater than the threshold thickness, or determine the amount of unit orthodontic movement of the specific tooth so that the orthodontic period falls within the allowable orthodontic range. However, the present invention is not limited thereto.

Thereafter, the computing device 100 may establish the orthodontic planning including the information on the amount of unit orthodontic movement for at least one tooth and the orthodontic period expected to be required for correction according to the amount of unit orthodontic movement, and provide the information on the established orthodontic planning as the solution for establishing orthodontic planning.

In various embodiments, the computing device 100 may simulate the correction for at least one tooth according to the orthodontic planning through the 3D tooth model, and provide the results of the simulation together with the information on the orthodontic planning as the solution for establishing orthodontic planning.

Here, various technologies known for performing the orthodontic planning based on the 3D tooth model may be selectively used, and the present specification does not limit the specific details of the simulation operation performed by the computing device. Hereinafter, a method of generating a 3D tooth model will be described with reference to FIGS. 4 to 6.

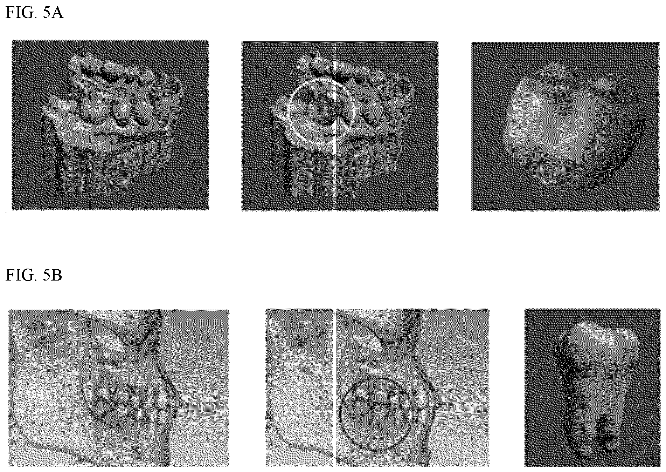

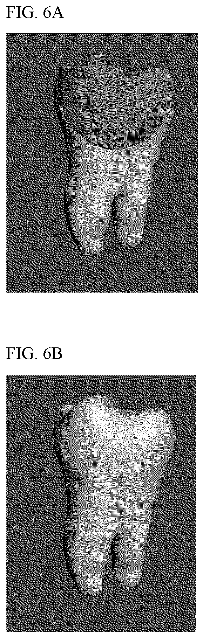

FIG. 4 is a flowchart for describing a method of generating a 3D tooth model in various embodiments, FIGS. 5A and 5B are diagrams illustrating tooth scan data and tooth CT data used for generating a 3D tooth model in various embodiments, and FIGS. 6A and 6B are diagrams for exemplarily illustrating a form in which the tooth scan data and the tooth CT data are aligned in various embodiments.

Referring to FIGS. 4 to 6, in operation S210, the computing device 100 may segment a plurality of first areas corresponding to each of the plurality of teeth from the tooth scan data (e.g., FIG. 5A). For example, the computing device 100 may set an area of interest (ROI) corresponding to each of the plurality of teeth in the tooth scan data, and segment and extract the plurality of first areas using the set area of interest.

In this case, the computing device 100 may configure polygon data using the tooth scan data, and segment and extract the plurality of first areas in the form of polygon data by performing polygon data segmentation.

In operation S220, the computing device 100 may segment a plurality of second areas corresponding to each of the plurality of teeth from the tooth CT data (e.g., FIG. 5B). For example, the computing device 100 may set an ROI corresponding to each of the plurality of teeth in the tooth CT data, and segment and extract the plurality of second areas using the set area of interest.

In this case, the computing device 100 may perform the 3D image data segmentation considering the dental CT data as 3D image data, thereby segmenting and extracting the plurality of second areas in the form of the 3D image.

In operation S230, the computing device 100 may generate a plurality of individual tooth models by registering the plurality of first areas and the plurality of second areas (e.g., FIGS. 6A and 6B).

In various embodiments, the computing device 100 may perform image to image registration on the plurality of first areas and the plurality of second areas. For example, the computing device 100 may convert the plurality of first areas extracted in the form of the polygon data into the form of the image data, and register the plurality of first areas converted into the form of the image data and the plurality of second areas extracted in the form of image data (e.g., at least one of landmark-based registration and/or voxel-based registration).

In various embodiments, the computing device 100 may perform polygon to polygon registration on the plurality of first areas and the plurality of second areas. For example, the computing device 100 may convert the plurality of second areas extracted in the form of the image data into a polygonal form, and may register (e.g., at least one of landmark-based registration and/or voxel-based registration) the plurality of second areas converted into the form of the polygon data and the plurality of first areas extracted in the form of the polygon data.

In various embodiments, the computing device 100 may replace the crown area included in the plurality of second areas with the crown area included in the plurality of first areas, considering that the tooth scan data has higher accuracy for the crown area than the tooth CT data. Through this, it is possible to perform more accurate modeling of not only the crown but also the root area.

In operation S240, the computing device 100 may generate a 3D tooth model by arranging each of the plurality of individual tooth models in the position of the tooth corresponding to each of the plurality of individual tooth models.

A method of providing a solution for establishing orthodontic planning using a 3D tooth model has been described above with reference to the flowchart illustrated in the drawing. For a simple explanation, the method of providing a solution for establishing orthodontic planning using a 3D tooth model has been described by showing a series of blocks, but the present invention is not limited to the order of the blocks, and some blocks may be performed in an order different from that shown and performed in the present specification, or may be performed concurrently. In addition, new blocks not described in the present specification and drawings may be added, or some blocks may be deleted or changed.

According to various embodiments of the present invention, it is possible to more accurately establish the orthodontic planning by generating the 3D tooth model by performing the accurate modeling on not only the crown area but also the root area and establishing the orthodontic planning using the generated 3D tooth model.

Effects of the present invention are not limited to the effects described above, and other effects that are not mentioned may be obviously understood by those skilled in the art from the following description.

Although the embodiments of the present invention have been described with reference to the accompanying drawings, those skilled in the art will understand that various modifications and alterations may be made without departing from the scope of the present invention as defined in the appended claims.

## Claims

1. A method of providing a solution for establishing orthodontic planning using a 3D tooth model by a computing device, the method comprising:
generating the 3D tooth model including a plurality of individual tooth models generated by individually modeling each of a plurality of teeth for a target (S110); and
providing the solution for establishing orthodontic planning based on the generated 3D tooth model (S120),
**characterized in that** the providing of the solution includes:
establishing temporary orthodontic planning for the at least one tooth based on a crown area of at least one individual tooth model corresponding to at least one of the plurality of teeth that requires correction;
reviewing the established temporary orthodontic planning based on a root area of the at least one individual tooth model; and
providing a solution for establishing final orthodontic planning based on the review result of the established temporary orthodontic planning.

2. The method of claim 1, wherein the reviewing of the established temporary orthodontic planning includes:
calculating an expected amount of orthodontic movement for the root area of the at least one tooth corresponding to an amount of unit orthodontic movement based on the amount of unit orthodontic movement for the at least one tooth according to the established temporary orthodontic planning, the amount of unit orthodontic movement including a unit movement amount and a unit rotation amount in a direction based on at least one of an X-axis, a Y-axis, and a Z-axis; and
determining whether the calculated expected correction amount is greater than or equal to a threshold value, and
the providing of the solution for establishing the final orthodontic planning includes providing information on the calculated expected amount of orthodontic movement, and when the calculated expected amount of orthodontic movement is greater than or equal to the threshold value, guiding adjustment of the amount of unit orthodontic movement according to the calculated expected amount of orthodontic movement or automatically adjusting the amount of unit orthodontic movement according to the calculated expected amount of orthodontic movement.

3. The method of claim 2, wherein the calculating of the expected amount of orthodontic movement for the root area of the at least one tooth includes:
setting an axis in a longitudinal direction of the tooth on the root area of the at least one individual tooth model;
setting a center of movement on the root area of the at least one individual tooth model; and
calculating the expected amount of orthodontic movement for the root area of the at least one tooth corresponding to the amount of unit orthodontic movement using the set axis and the set center of movement.

4. The method of claim 3, wherein the setting of the center of movement includes setting the center of movement on the root area of the at least one individual tooth model using at least one of a method of setting the center of movement based on a user input acquired through a user interface (UI) that outputs the generated 3D tooth model and a method of automatically setting the center of movement based on a landmark located on the root area of the at least one individual tooth model.

5. The method of claim 1, wherein the reviewing of the established temporary orthodontic planning includes:
determining an expected movement direction and an expected movement amount of a root area of a specific tooth when a crown area of the specific tooth moves in a specific direction according to the established temporary orthodontic planning; and
determining a position of the root area of the specific tooth based on the determined expected movement direction and the determined expected movement amount.

6. The method of claim 1, wherein the reviewing of the established temporary orthodontic planning includes:
calculating an orthodontic amount of the root area relative to an orthodontic amount of the crown area for each of the plurality of individual tooth models using the generated 3D tooth model; and
calculating an orthodontic period for the at least one tooth based on the calculated orthodontic amount of the root area relative to the orthodontic amount of the crown area, and
the providing of the solution for establishing the final orthodontic planning includes providing information on the calculated orthodontic period.

7. The method of claim 6, wherein the calculating of the orthodontic period includes:
when correction from a first point to a second point is required for a specific tooth, dividing a section from the first point to the second point based on the amount of unit orthodontic movement for the specific tooth according to the established temporary orthodontic planning; and
calculating the orthodontic period for the at least one tooth based on the number of divided sections.

8. The method of claim 1, wherein the providing of the solution includes: when movement from a current position to a target position is required for at least one of the plurality of teeth, determining an amount of unit orthodontic movement for the at least one tooth using at least one individual tooth model corresponding to each of the at least one tooth; and
establishing orthodontic planning including information on the determined amount of unit orthodontic movement and the orthodontic period expected to be required when correcting the at least one tooth according to the determined amount of unit orthodontic movement, and providing information on the established orthodontic planning.

9. The method of claim 8, wherein the providing of the information on the established orthodontic planning includes simulating the correction of the at least one tooth according to the established orthodontic planning through the generated 3D tooth model, and providing a result of the simulation together with the information on the established orthodontic planning.

10. The method of claim 8, wherein the generating of the 3D tooth model includes:
dividing a plurality of first areas corresponding to each of the plurality of teeth from tooth scan data;
dividing a plurality of second areas corresponding to each of the plurality of teeth from tooth scan data; and
generating a plurality of individual tooth models by aligning the plurality of divided first areas with the plurality of divided second areas, and
generating the 3D tooth model by arranging each of the plurality of generated individual tooth models at a position of a tooth corresponding to each of the plurality of generated individual tooth models.

11. A computing device (100) for performing a method of providing a solution for establishing orthodontic planning using a 3D tooth model, the computing device comprising:
a processor (110);
a network interface (140);
a memory (120); and
a computer program (151) loaded into the memory and executed by the processor,
wherein the computer program includes:
an instruction for generating the 3D tooth model including a plurality of individual tooth models generated by individually modeling each of a plurality of teeth for a target; and
an instruction for providing the solution for establishing orthodontic planning based on the generated 3D tooth model,
**characterized in that** the providing of the solution includes:
establishing temporary orthodontic planning for the at least one tooth based on a crown area of at least one individual tooth model corresponding to at least one of the plurality of teeth that requires correction;
reviewing the established temporary orthodontic planning based on a root area of the at least one individual tooth model; and
providing a solution for establishing final orthodontic planning based on the review result of the established temporary orthodontic planning.

12. A computer program (151) stored on a computing device-readable recording medium, coupled to the computing device to perform a method of providing a solution for establishing orthodontic planning using a 3D tooth model, wherein the computer program includes the following operations:
generating the 3D tooth model including a plurality of individual tooth models generated by individually modeling each of a plurality of teeth for a target; and
providing the solution for establishing orthodontic planning based on the generated 3D tooth model,
**characterized in that** the providing of the solution includes:
establishing temporary orthodontic planning for the at least one tooth based on a crown area of at least one individual tooth model corresponding to at least one of the plurality of teeth that requires correction;
reviewing the established temporary orthodontic planning based on a root area of the at least one individual tooth model; and
providing a solution for establishing final orthodontic planning based on the review result of the established temporary orthodontic planning.

## Patentansprüche

1. Verfahren zum Bereitstellen einer Lösung zur Herstellung einer orthodontischen Planung unter Verwendung eines 3D-Zahnmodells durch eine Rechnervorrichtung, wobei das Verfahren Folgendes umfasst:
Erzeugen des 3D-Zahnmodells, das eine Vielzahl von einzelnen Zahnmodellen beinhaltet, die durch einzelnes Modellieren von jedem aus einer Vielzahl von Zähnen für ein Ziel (S110) erzeugt werden; und
Bereitstellen der Lösung zur Herstellung einer orthodontischen Planung basierend auf dem erzeugten 3D-Zahnmodell (S120),
**dadurch gekennzeichnet, dass** das Bereitstellen der Lösung Folgendes beinhaltet:
Herstellen einer temporären orthodontischen Planung für den zumindest einen Zahn basierend auf einem Kronenbereich von zumindest einem einzelnen Zahnmodell entsprechend zumindest einem aus der Vielzahl von Zähnen, der Korrektur erfordert;
Überprüfen der hergestellten temporären orthodontischen Planung basierend auf einem Wurzelbereich des zumindest einen einzelnen Zahnmodells; und
Bereitstellen einer Lösung zur Herstellung einer finalen orthodontischen Planung basierend auf dem Überprüfungsergebnis der hergestellten temporären orthodontischen Planung.

2. Verfahren nach Anspruch 1, wobei das Überprüfen der hergestellten temporären orthodontischen Planung Folgendes beinhaltet:
Berechnen einer erwarteten Menge an orthodontischer Bewegung für den Wurzelbereich des zumindest einen Zahns entsprechend einer Menge an orthodontischer Einheitsbewegung basierend auf der Menge an orthodontischer Einheitsbewegung für den zumindest einen Zahn gemäß der hergestellten temporären orthodontischen Planung, wobei die Menge an orthodontischer Einheitsbewegung eine Einheitsbewegungsmenge und eine Einheitsdrehungsmenge in einer Richtung basierend auf zumindest einem von einer X-Achse, einer Y-Achse und einer Z-Achse beinhaltet; und
Bestimmen, ob die berechnete erwartete Korrekturmenge größer als ein oder gleich einem Schwellenwert ist, und
das Bereitstellen der Lösung zur Herstellung der finalen orthodontischen Planung Bereitstellen von Informationen über die berechnete erwartete Menge an orthodontischer Bewegung, und wenn die berechnete erwartete Menge an orthodontischer Bewegung größer als der oder gleich dem Schwellenwert ist, Führen von Anpassung der Menge an orthodontischer Einheitsbewegung gemäß der berechneten erwarteten Menge an orthodontischer Bewegung oder automatisches Anpassen der Menge an orthodontischer Einheitsbewegung gemäß der berechneten erwarteten Menge an orthodontischer Bewegung beinhaltet.

3. Verfahren nach Anspruch 2, wobei das Berechnen der erwarteten Menge an orthodontischer Bewegung für den Wurzelbereich des zumindest einen Zahns Folgendes beinhaltet:
Festlegen einer Achse in einer Längsrichtung des Zahns an dem Wurzelbereich des zumindest einen einzelnen Zahnmodells;
Festlegen eines Bewegungszentrums auf dem Wurzelbereich des zumindest einen einzelnen Zahnmodells; und
Berechnen der erwarteten Menge an orthodontischer Bewegung für den Wurzelbereich des zumindest einen Zahns entsprechend der Menge an orthodontischer Einheitsbewegung unter Verwendung der festgelegten Achse und des festgelegten Bewegungszentrums.

4. Verfahren nach Anspruch 3, wobei das Festlegen des Bewegungszentrums Festlegen des Bewegungszentrums an dem Wurzelbereich des zumindest einen einzelnen Zahnmodells unter Verwendung von zumindest einem von einem Verfahren zum Festlegen des Bewegungszentrums basierend auf einer Benutzereingabe, die durch eine Benutzerschnittstelle (UI) erfasst wird, die das erzeugte 3D-Zahnmodell ausgibt, und einem Verfahren zum automatischen Festlegen des Bewegungszentrums basierend auf einem Orientierungspunkt, der sich an dem Wurzelbereich des zumindest einen einzelnen Zahnmodells befindet, beinhaltet.

5. Verfahren nach Anspruch 1, wobei das Überprüfen der hergestellten temporären orthodontischen Planung Folgendes beinhaltet:
Bestimmen einer erwarteten Bewegungsrichtung und einer erwarteten Bewegungsmenge eines Wurzelbereichs eines spezifischen Zahns, wenn sich ein Kronenbereich des spezifischen Zahns in einer spezifischen Richtung gemäß der hergestellten temporären orthodontischen Planung bewegt; und
Bestimmen einer Position des Wurzelbereichs des spezifischen Zahns basierend auf der bestimmten erwarteten Bewegungsrichtung und der bestimmten erwarteten Bewegungsmenge.

6. Verfahren nach Anspruch 1, wobei das Überprüfen der hergestellten temporären orthodontischen Planung Folgendes beinhaltet:
Berechnen einer orthodontischen Menge des Wurzelbereichs relativ zu einer orthodontischen Menge des Kronenbereichs für jedes aus der Vielzahl von einzelnen Zahnmodellen unter Verwendung des erzeugten 3D-Zahnmodells; und
Berechnen eines orthodontischen Zeitraums für den zumindest einen Zahn basierend auf der berechneten orthodontischen Menge des Wurzelbereichs relativ zu der orthodontischen Menge des Kronenbereichs, und
das Bereitstellen der Lösung zur Herstellung der finalen orthodontischen Planung Bereitstellen von Informationen über den berechneten orthodontischen Zeitraum beinhaltet.

7. Verfahren nach Anspruch 6, wobei das Berechnen des orthodontischen Zeitraums Folgendes beinhaltet:
wenn Korrektur von einem ersten Punkt zu einem zweiten Punkt für einen spezifischen Zahn erforderlich ist, Unterteilen eines Abschnittes von dem ersten Punkt zu dem zweiten Punkt basierend auf der Menge an orthodontischer Einheitsbewegung für den spezifischen Zahn gemäß der hergestellten temporären orthodontischen Planung; und
Berechnen des orthodontischen Zeitraums für den zumindest einen Zahn basierend auf der Anzahl an unterteilten Abschnitten.

8. Verfahren nach Anspruch 1, wobei das Bereitstellen der Lösung Folgendes beinhaltet: wenn Bewegung von einer aktuellen Position zu einer Zielposition für zumindest einen aus der Vielzahl von Zähnen erforderlich ist, Bestimmen einer Menge an orthodontischer Einheitsbewegung für den zumindest einen Zahn unter Verwendung von zumindest einem einzelnen Zahnmodell entsprechend jedem von dem zumindest einen Zahn; und
Herstellen einer orthodontischen Planung, die Informationen über die bestimmte Menge an orthodontischer Einheitsbewegung und den orthodontischen Zeitraum, der erwarteterweise erforderlich ist, wenn der zumindest eine Zahn gemäß der bestimmten Menge an orthodontischer Einheitsbewegung korrigiert wird, und Bereitstellen von Informationen über die hergestellte orthodontische Planung beinhaltet.

9. Verfahren nach Anspruch 8, wobei das Bereitstellen der Informationen über die hergestellte orthodontische Planung Simulieren der Korrektur des zumindest einen Zahns gemäß der hergestellten orthodontischen Planung durch das erzeugte 3D-Zahnmodell und Bereitstellen eines Ergebnisses der Simulation zusammen mit den Informationen über die hergestellte orthodontische Planung beinhaltet.

10. Verfahren nach Anspruch 8, wobei das Erzeugen des 3D-Zahnmodells Folgendes beinhaltet:
Unterteilen einer Vielzahl von ersten Bereichen entsprechend jedem aus der Vielzahl von Zähnen aus Zahnabtastdaten;
Unterteilen einer Vielzahl von zweiten Bereichen entsprechend jedem aus der Vielzahl von Zähnen aus Zahnabtastdaten; und
Erzeugen einer Vielzahl von einzelnen Zahnmodellen durch Ausrichten der Vielzahl von unterteilten ersten Bereichen mit der Vielzahl von unterteilten zweiten Bereichen, und
Erzeugen des 3D-Zahnmodells durch Anordnen von jedem aus der Vielzahl von erzeugten einzelnen Zahnmodellen an einer Position eines Zahns entsprechend jedem aus der Vielzahl von erzeugten einzelnen Zahnmodellen.

11. Rechnervorrichtung (100) zum Durchführen eines Verfahrens zum Bereitstellen einer Lösung zur Herstellung einer orthodontischen Planung unter Verwendung eines 3D-Zahnmodells, wobei die Rechnervorrichtung Folgendes umfasst:
einen Prozessor (110);
eine Netzwerkschnittstelle (140);
einen Speicher (120); und
ein Computerprogramm (151), das in den Speicher geladen und durch den Prozessor ausgeführt wird,
wobei das Computerprogramm Folgendes beinhaltet:
eine Anweisung zum Erzeugen des 3D-Zahnmodells, das eine Vielzahl von einzelnen Zahnmodellen beinhaltet, die durch einzelnes Modellieren von jedem aus einer Vielzahl von Zähnen für ein Ziel erzeugt werden; und
eine Anweisung zur Bereitstellung der Lösung zur Herstellung einer orthodontischen Planung basierend auf dem erzeugten 3D-Zahnmodell,
**dadurch gekennzeichnet, dass** das Bereitstellen der Lösung Folgendes beinhaltet:
Herstellen einer temporären orthodontischen Planung für den zumindest einen Zahn basierend auf einem Kronenbereich von zumindest einem einzelnen Zahnmodell entsprechend zumindest einem aus der Vielzahl von Zähnen, der Korrektur erfordert;
Überprüfen der hergestellten temporären orthodontischen Planung basierend auf einem Wurzelbereich des zumindest einen einzelnen Zahnmodells; und
Bereitstellen einer Lösung zur Herstellung einer finalen orthodontischen Planung basierend auf dem Überprüfungsergebnis der hergestellten temporären orthodontischen Planung.

12. Computerprogramm (151), das auf einem von einer Rechnervorrichtung lesbaren Aufzeichnungsmedium gespeichert ist, das an die Rechnervorrichtung gekoppelt ist, um ein Verfahren zum Bereitstellen einer Lösung zur Herstellung einer orthodontischen Planung unter Verwendung eines 3D-Zahnmodells durchzuführen, wobei das Computerprogramm die folgenden Operationen beinhaltet:
Erzeugen des 3D-Zahnmodells, das eine Vielzahl von einzelnen Zahnmodellen beinhaltet, die durch einzelnes Modellieren von jedem aus einer Vielzahl von Zähnen für ein Ziel erzeugt werden; und
Bereitstellen der Lösung zur Herstellung einer orthodontischen Planung basierend auf dem erzeugten 3D-Zahnmodell,
**dadurch gekennzeichnet, dass** das Bereitstellen der Lösung Folgendes beinhaltet:
Herstellen einer temporären orthodontischen Planung für den zumindest einen Zahn basierend auf einem Kronenbereich von zumindest einem einzelnen Zahnmodell entsprechend zumindest einem aus der Vielzahl von Zähnen, der Korrektur erfordert;
Überprüfen der hergestellten temporären orthodontischen Planung basierend auf einem Wurzelbereich des zumindest einen einzelnen Zahnmodells; und
Bereitstellen einer Lösung zur Herstellung einer finalen orthodontischen Planung basierend auf dem Überprüfungsergebnis der hergestellten temporären orthodontischen Planung.

## Revendications

1. Procédé de fourniture d'une solution pour établir une planification orthodontique à l'aide d'un modèle dentaire 3D par un dispositif informatique, le procédé comprenant :
la génération du modèle dentaire 3D comprenant une pluralité de modèles dentaires individuels générés en modélisant individuellement chacune d'une pluralité de dents pour une cible (S110) ;
et la fourniture de la solution pour établir une planification orthodontique sur la base du modèle dentaire 3D généré (S120),
**caractérisé en ce que** la fourniture de la solution comprend :
l'établissement d'une planification orthodontique temporaire pour l'au moins une dent sur la base d'une zone de couronne d'au moins un modèle dentaire individuel correspondant à au moins l'une de la pluralité de dents qui nécessite une correction ;
l'examen de la planification orthodontique temporaire établie sur la base d'une zone de racine du au moins un modèle dentaire individuel ; et
la fourniture d'une solution pour établir une planification orthodontique finale sur la base du résultat d'examen de la planification orthodontique temporaire établie.

2. Procédé de la revendication 1, ledit examen de la planification orthodontique temporaire établie comprenant :
le calcul d'une quantité attendue de mouvement orthodontique pour la zone de racine de l'au moins une dent correspondant à une quantité de mouvement orthodontique unitaire sur la base de la quantité de mouvement orthodontique unitaire pour l'au moins une dent selon la planification orthodontique temporaire établie, la quantité de mouvement orthodontique unitaire comprenant une quantité de mouvement unitaire et une quantité de rotation unitaire dans une direction sur la base d'au moins l'un d'un axe X, d'un axe Y et d'un axe Z ; et
la détermination pour savoir si la quantité de correction attendue calculée est supérieure ou égale à une valeur seuil, et
la fourniture de la solution pour établir la planification orthodontique finale comprend la fourniture d'informations sur la quantité prévue calculée de mouvement orthodontique, et lorsque la quantité prévue calculée de mouvement orthodontique est supérieure ou égale à la valeur seuil, le guidage du réglage de la quantité de mouvement orthodontique unitaire selon la quantité prévue calculée de mouvement orthodontique ou le réglage automatique de la quantité de mouvement orthodontique unitaire selon la quantité prévue calculée de mouvement orthodontique.

3. Procédé de la revendication 2, ledit calcul de la quantité attendue de mouvement orthodontique pour la zone de racine de l'au moins une dent comprenant :
la définition d'un axe dans une direction longitudinale de la dent sur la zone de racine du au moins un modèle dentaire individuel ;
la définition d'un centre de mouvement sur la zone de racine du au moins un modèle dentaire individuel ; et
le calcul de la quantité attendue de mouvement orthodontique pour la zone de racine de l'au moins une dent correspondant à la quantité de mouvement orthodontique unitaire à l'aide de l'axe défini et du centre de mouvement défini.

4. Procédé de la revendication 3, ledit réglage du centre de mouvement comprenant le réglage du centre de mouvement sur la zone de racine du au moins un modèle dentaire individuel à l'aide d'au moins l'un d'un procédé de définition du centre de mouvement sur la base d'une entrée utilisateur acquise par l'intermédiaire d'une interface utilisateur (UI) qui délivre en sortie le modèle dentaire 3D généré et d'un procédé de définition automatique du centre de mouvement sur la base d'un point de repère situé sur la zone de racine du au moins un modèle dentaire individuel.

5. Procédé de la revendication 1, ledit examen de la planification orthodontique temporaire établie comprenant :
la détermination d'une direction de mouvement attendue et d'une quantité de mouvement attendue d'une zone de racine d'une dent spécifique lorsqu'une zone de couronne de la dent spécifique se déplace dans une direction spécifique selon la planification orthodontique temporaire établie ; et
la détermination d'une position de la zone de racine de la dent spécifique sur la base de la direction de mouvement attendue déterminée et de la quantité de mouvement attendue déterminée.

6. Procédé de la revendication 1, ledit examen de la planification orthodontique temporaire établie comprenant :
le calcul d'une quantité orthodontique de la zone de racine par rapport à une quantité orthodontique de la zone de couronne pour chacun de la pluralité de modèles dentaires individuels à l'aide du modèle dentaire 3D généré ; et
le calcul d'une période orthodontique pour l'au moins une dent sur la base de la quantité orthodontique calculée de la zone de racine par rapport à la quantité orthodontique de la zone de couronne, et
la fourniture de la solution pour établir la planification orthodontique finale comprend la fourniture d'informations sur la période orthodontique calculée.

7. Procédé de la revendication 6, ledit calcul de la période orthodontique comprenant :
lorsqu'une correction d'un premier point vers un second point est nécessaire pour une dent spécifique, la division d'une section du premier point vers le second point sur la base de la quantité de mouvement orthodontique unitaire pour la dent spécifique selon la planification orthodontique temporaire établie ; et
le calcul de la période orthodontique pour l'au moins une dent sur la base du nombre de sections divisées.

8. Procédé de la revendication 1, ladite fourniture de la solution comprenant : lorsque le mouvement d'une position actuelle à une position cible est nécessaire pour au moins l'une de la pluralité de dents, la détermination d'une quantité de mouvement orthodontique unitaire pour l'au moins une dent à l'aide d'au moins un modèle dentaire individuel correspondant à chacune de l'au moins une dent ; et
l'établissement d'une planification orthodontique comprenant des informations sur la quantité déterminée de mouvement orthodontique unitaire et la période orthodontique qui devrait être nécessaire lors de la correction de l'au moins une dent selon la quantité déterminée de mouvement orthodontique unitaire, et la fourniture d'informations sur la planification orthodontique établie.

9. Procédé de la revendication 8, ladite fourniture des informations sur la planification orthodontique établie comprenant la simulation de la correction de l'au moins une dent selon la planification orthodontique établie à travers le modèle dentaire 3D généré, et la fourniture d'un résultat de la simulation avec les informations sur la planification orthodontique établie.

10. Procédé de la revendication 8, ladite génération du modèle dentaire 3D comprenant :
la division d'une pluralité de premières zones correspondant à chacune de la pluralité de dents à partir de données de balayage de dent ;
la division d'une pluralité de secondes zones correspondant à chacune de la pluralité de dents à partir de données de balayage de dent ; et
la génération d'une pluralité de modèles dentaires individuels en alignant la pluralité de premières zones divisées avec la pluralité de secondes zones divisées, et
la génération du modèle dentaire 3D en agençant chacun de la pluralité de modèles dentaires individuels générés au niveau d'une position d'une dent correspondant à chacun de la pluralité de modèles dentaires individuels générés.

11. Dispositif informatique (100) destiné à réaliser un procédé de fourniture d'une solution pour établir une planification orthodontique à l'aide d'un modèle dentaire 3D, le dispositif informatique comprenant :
un processeur (110) ;
une interface réseau (140) ;
une mémoire (120) ; et
un programme informatique (151) chargé dans la mémoire et exécuté par le processeur,
ledit programme informatique comprenant :
une instruction pour générer le modèle dentaire 3D comprenant une pluralité de modèles dentaires individuels générés en modélisant individuellement chacune d'une pluralité de dents pour une cible ; et
une instruction pour fournir la solution pour établir une planification orthodontique sur la base du modèle dentaire 3D généré,
**caractérisé en ce que** la fourniture de la solution comprend :
l'établissement d'une planification orthodontique temporaire pour l'au moins une dent sur la base d'une zone de couronne d'au moins un modèle dentaire individuel correspondant à au moins l'une de la pluralité de dents qui nécessite une correction ;
l'examen de la planification orthodontique temporaire établie sur la base d'une zone de racine du au moins un modèle dentaire individuel ; et
la fourniture d'une solution pour établir une planification orthodontique finale sur la base du résultat d'examen de la planification orthodontique temporaire établie.

12. Programme informatique (151) stocké sur un support d'enregistrement lisible par un dispositif informatique, couplé au dispositif informatique pour réaliser un procédé de fourniture d'une solution pour établir une planification orthodontique à l'aide d'un modèle dentaire 3D, ledit programme informatique comprenant les opérations suivantes :
la génération du modèle dentaire 3D comprenant une pluralité de modèles dentaires individuels générés en modélisant individuellement chacune d'une pluralité de dents pour une cible ; et
la fourniture de la solution pour établir une planification orthodontique sur la base du modèle dentaire 3D généré,
**caractérisé en ce que** la fourniture de la solution comprend :
l'établissement d'une planification orthodontique temporaire pour l'au moins une dent sur la base d'une zone de couronne d'au moins un modèle dentaire individuel correspondant à au moins l'une de la pluralité de dents qui nécessite une correction ;
l'examen de la planification orthodontique temporaire établie sur la base d'une zone de racine du au moins un modèle dentaire individuel ; et
la fourniture d'une solution pour établir une planification orthodontique finale sur la base du résultat d'examen de la planification orthodontique temporaire établie.
